# EUROPEAN PATENT SPECIFICATION

(11) **EP 2 897 579 B1**
(45) Date of publication and mention of the grant of the patent: **21.02.2018**
(21) Application number: 13771720.3
(22) Date of filing: 20.09.2013
(51) Int. Cl.: A61K 8/37, A61K 8/58, A61Q 13/00, A61K 8/89, A61K 8/02, A61K 8/11, A61K 8/81, A61Q 15/00

(54) **COMPOSITIONS AND ARTICLES HAVING A PARENT FRAGRANCE AND MICROCAPSULES ENCAPSULATING A NON-PARENT FRAGRANCE**
ZUSAMMENSETZUNGEN UND ARTIKEL MIT EINEM PARENT-DUFTSTOFF UND MIKROKAPSELN MIT EINEM NON-PARENT-DUFTSTOFF
COMPOSITIONS ET ARTICLES PRÉSENTANT UNE FRAGRANCE MÈRE ET DES MICROCAPSULES ENCAPSULANT UNE AUTRE FRAGRANCE NON MÈRE

(30) Priority: 20.09.2012 US 201261703587 P; 20.09.2012 US 201261703616 P
(43) Date of publication of application: 29.07.2015
(73) Proprietor: The Procter & Gamble Company, Cincinnati, OH 45202 (US)
(72) Inventor: GHOSH, Chanchal Kumar, Cincinnati, Ohio 45202 (US); ZIMMERMAN, Dean Arthur, Cincinnati, Ohio 45202 (US); LI, Jianjun Justin, Cincinnati, OH 45202 (US); DIHORA, Jiten Odhavji, Cincinnati, OH 45202 (US); CETTI, Jonathan Robert, Cincinnati, OH 45202 (US); WITT, Steven Edward, Cincinnati, OH 45202 (US); HUTCHINS, Virginia Tzung-hwei, Cincinnati, OH 45202 (US); FLICKINGER, Marc Adam, Cincinnati, OH 45202 (US); LABITZKE, Kevin Max, Cincinnati, OH 45202 (US); DUBOIS, Zerlina Guzdar, Cincinnati, OH 45202 (US)
(74) Representative: Sauvaître, Thibault Bruno
(86) International application number: PCT/US2013/061030
(87) International publication number: WO 2014/047507

(56) References cited:
- EP-A2- 2 433 617
- US-A1- 2007 248 553
- US-A1- 2010 104 612
- US-A1- 2012 076 839
- US-A1- 2012 121 677

## Description

### FIELD

The present disclosure generally relates to compositions with fragrances, and specifically relates to compositions having a parent fragrance and microcapsules encapsulating a non-parent fragrance.

### BACKGROUND

Some personal care products, such as antiperspirants and deodorants, are designed to provide the user with protection from wetness and/or malodor. Such products often include compositions with a parent fragrance dispersed throughout the composition. These compositions may also include a moisture triggered fragrance delivery technology, such as fragrance loaded beta-cyclodextrin or starch encapsulated accords, which can provide a burst of fragrance when triggered by a threshold level of moisture (e.g. sweat). However, a composition with a moisture triggered fragrance technology may not always receive enough moisture to be triggered. For example, a user may not sweat enough to trigger the release of a noticeable burst of fragrance, or an antiperspirant may reduce sweat to such a low level that the sweat does not trigger the release of a noticeable burst of fragrance.

U.S. Patent Application 2012/076839 A1 is related to an anhydrous antiperspirant composition containing an encapsulated fragrance, in which the encapsulate shell comprises an aminoplast resin, and preferably a melamine/formaldehyde resin.

U.S. Patent Application 2010/104612 A1 is related to an anhydrous antiperspirant composition comprising particulate antiperspirant active, water-insoluble, dry particulate friction-sensitive capsules of perfume, and a liquid carrier for the particulate antiperspirant active and capsules of perfume comprising at least one water-immiscible oil.

U.S. Patent Application 2007/0248553 A1 concerns antiperspirant compositions comprising: (a) from 0.1% to 30% by weight of the composition, of a high-efficacy antiperspirant active; (b) from 0.1% to 35% by weight of the composition, of a thickening agent; (c) from 10% to 99% by weight of the composition, of an anhydrous liquid carrier; (d) from 5 ppm to 20% by weight of the composition, of a primary fragrance; and (e) from at least 5 ppm by weight of the composition, of a secondary fragrance that is distinct from the primary fragrance and is included in a surfactant-free, water-releasable matrix.

U.S. Patent Application 2012/0121677 A1 refers to an antiperspirant or deodorant composition comprising i) an antiperspirant or deodorant active, ii) a liquid carrier for the antiperspirant or deodorant active, and iii) fragrance in which the fragrance comprises a mixture of first and second fragrances.

European Patent Application EP 2 433 617 A2 concerns a cosmetic composition comprising (a) microcapsules, whose capsule walls include a resin obtained by reacting (i) at least one aromatic alcohol or its ether or derivative, (ii) at least one aldehyde component having at least two carbon atoms per molecule, and (iii) optionally in the presence of at least one (meth)acrylate polymers; and (b) at least one cosmetic active ingredient.

Thus, there may be a need for a fragrance delivery system that allows for the delivery of a parent fragrance and a non-parent fragrance such that a consumer can notice the bloom of both fragrances.

### SUMMARY

An anhydrous antiperspirant composition comprising a parent fragrance that is dispersed throughout the composition; and a friction-triggered fragrance delivery technology comprising a plurality of microcapsules; wherein the microcapsules comprise a core material and a shell encapsulating the core material; wherein the core material comprises a first non-parent fragrance and the shell comprises a synthetic polymeric material, wherein the synthetic polymeric material comprises a polyacrylate material; further comprising a moisture-triggered fragrance delivery technology comprising a second non-parent fragrance; and wherein the second non-parent fragrance is encapsulated in cyclodextrin particles.

An anhydrous antiperspirant composition comprising: from 0.1 % to 30% by weight of the anhydrous composition, of one or more antiperspirant actives; from 0.1 % to 35% by weight of the anhydrous composition, of one or more structurants; from 10% to 99% by weight of the anhydrous composition, of one or more anhydrous liquid carriers; a parent fragrance that is dispersed throughout the anhydrous composition; and a friction-triggered fragrance delivery technology comprising a plurality of microcapsules; wherein the microcapsules comprise a core material and a shell encapsulating the core material; wherein the core material comprises a first non-parent fragrance and the shell comprises a synthetic polymeric material comprising a polyacrylate material; further comprising a moisture-triggered fragrance delivery technology comprising a second non-parent fragrance; wherein the second non-parent fragrance is encapsulated in cyclodextrin particles.

### BRIEF DESCRIPTION OF THE DRAWINGS

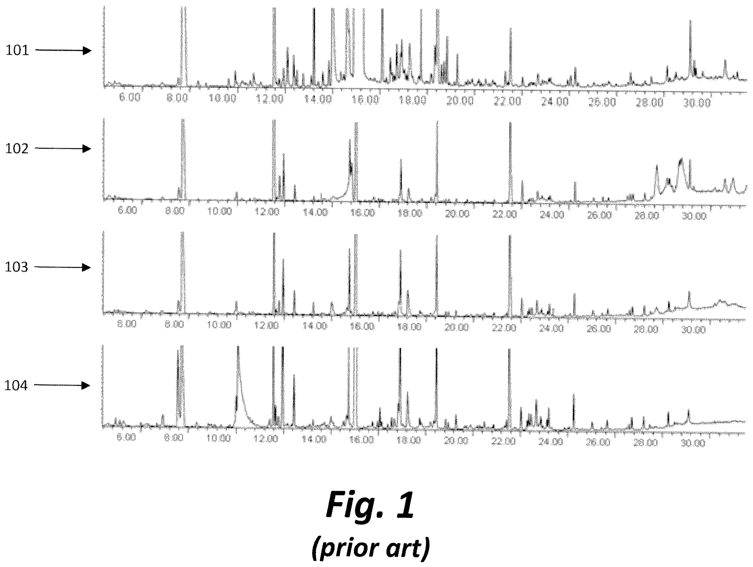

Figure 1 is a comparison of four GC-MS chromatograms obtained for a first Comparative Example, which is a deodorant/antiperspirant with parent fragrance, as known in the prior art.

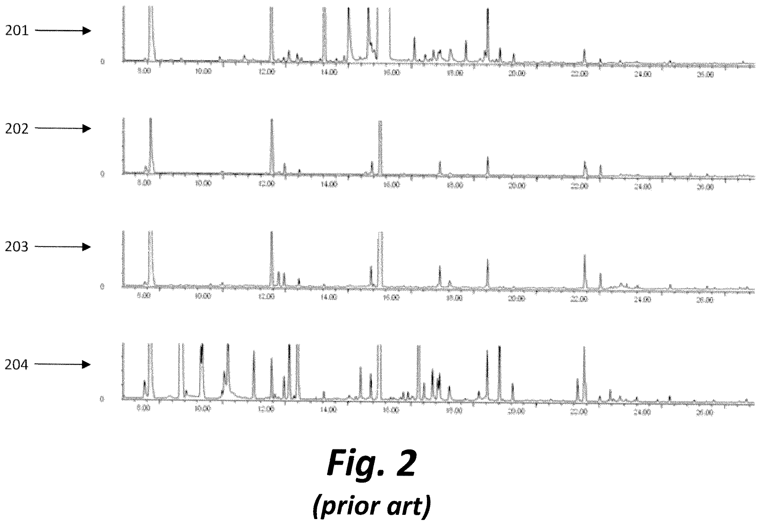

Figure 2 is a comparison of four GC-MS chromatograms obtained for a second Comparative Example, which is a deodorant/antiperspirant with parent fragrance and beta-cyclodextrin as a moisture triggered fragrance delivery technology, as known in the prior art.

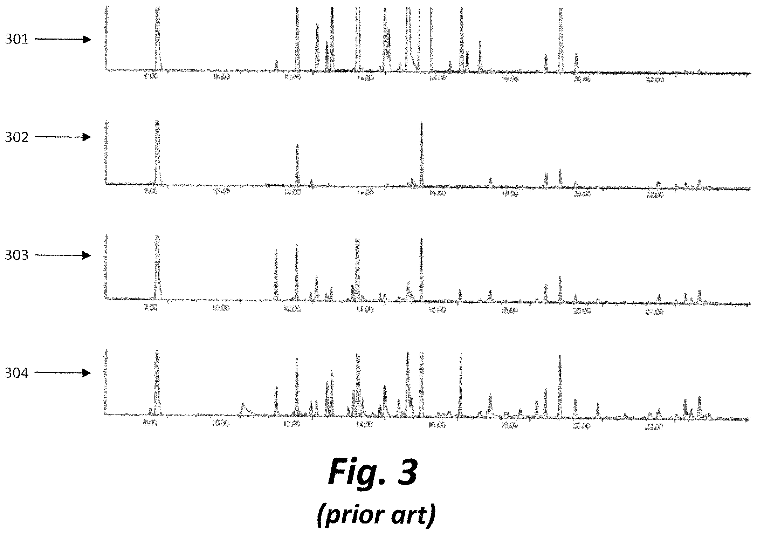

Figure 3 is a comparison of four GC-MS chromatograms obtained for a third Comparative Example, which is a deodorant/antiperspirant product with a parent fragrance, gelatin microcapsules as a friction triggered fragrance delivery technology for the same fragrance as the parent fragrance, and a starch encapsulated accord as a moisture triggered fragrance delivery technology for the same fragrance as the parent fragrance, as known in the prior art.

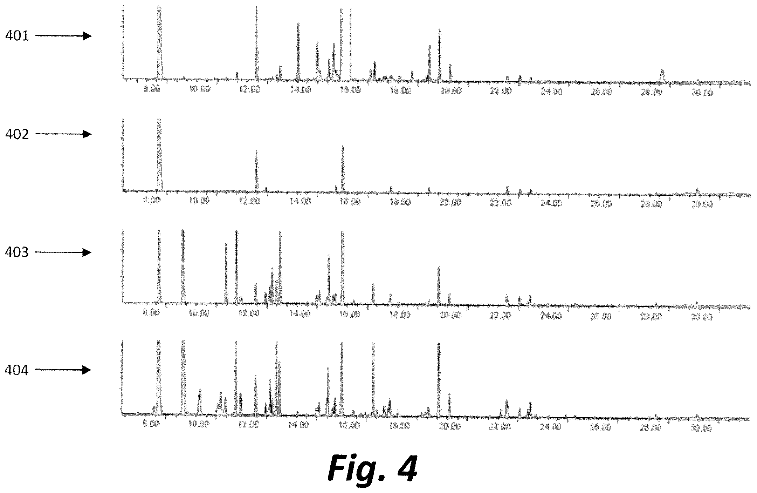

Figure 4 is a comparison of four GC-MS chromatograms obtained for a an Inventive Example, which is a deodorant/antiperspirant product with a parent fragrance, polyacrylate microcapsules as a friction triggered fragrance delivery technology for a first fragrance different from the parent fragrance, and beta-cyclodextrin as a moisture triggered fragrance delivery technology for a second fragrance different from the parent fragrance.

### DETAILED DESCRIPTION

The composition can be an anhydrous composition. The term "anhydrous" as used herein means that the antiperspirant stick composition of the present invention, and the essential or optional components thereof, are free of added or free water. From a formulation standpoint, this means that the anhydrous antiperspirant stick compositions of the present invention contain zero percent, by mass of free or added water, other than the water of hydration typically associated with the particulate antiperspirant active and/or the spray dried microcapsules prior to formulation. A parent fragrance may be a fragrance that is dispersed throughout the composition and is typically not encapsulated when added to the composition and/or article. Herein, a non-parent fragrance refers to a fragrance that differs from a parent fragrance included within the composition and/or article. Non-limiting examples of differences between a fragrance and a non-parent fragrance include differences in chemical make-up. Typically, a non-parent fragrance is encapsulated within a material before inclusion into a composition and/or article.

It has surprisingly been discovered that a friction-triggered fragrance technology can be added to a composition with a parent fragrance and a moisture-triggered fragrance technology. As a result, a bloom of fragrance can be delivered even when there is not enough moisture to trigger the release of the fragrance from the moisture-triggered fragrance technology. However, if the fragrance of the friction-triggered fragrance technology is the same as the parent fragrance, the release of the friction-triggered fragrance may not be noticeable to a user who has become habituated to the parent fragrance. Thus, the composition include a parent fragrance and a microcapsule encapsulated a non-parent fragrance.

The composition herein include microcapsules. The composition comprises a friction-triggered fragrance delivery technology comprising a plurality of microcapsules; wherein the microcapsules comprise a core material and a shell encapsulating the core material; wherein the core material comprises a first non-parent fragrance and the shell comprises a synthetic polymeric material comprising a polyacrylate material; further comprising a moisture-triggered fragrance delivery technology comprising a second non-parent fragrance; wherein the second non-parent fragrance is encapsulated in cyclodextrin particles.

Also as an example, the microcapsules can be friable microcapsules. A friable microcapsule is configured to release its core material when its shell is ruptured. The rupture can be caused by forces applied to the shell during mechanical interactions. Some or all of the friable microcapsules can have various fracture strengths. For at least a first group of the provided microcapsules, each microcapsule can have an outer shell with a fracture strength of 0.2-10.0 mega Pascals, when measured according to the Fracture Strength Test Method, or any incremental value expressed in 0.1 mega Pascals in this range, or any range formed by any of these values for fracture strength. As an example, a microcapsule can have an shell with a fracture strength of 0.2-2.0 mega Pascals.

Some or all of the microcapsules can have various core to shell ratios. For at least a first group of the provided microcapsules, each microcapsule can have a shell, a core within the shell, and a core to shell ratio that is greater than or equal to: 70% to 30%, 75% to 25%, 80% to 20%, 85% to 15%, 90% to 10%, 95% to 5%.

Some or all of the microcapsules can have shells made from any material in any size, shape, and configuration known in the art. Some or all of the shells can include a polyacrylate material, such as a polyacrylate random copolymer. In the claimed invention, the shell comprises a synthetic polymeric material comprising a polyacrylate material. For example, the polyacrylate random copolymer can have a total polyacrylate mass, which includes ingredients selected from the group including: amine content of 0.2-2.0% of total polyacrylate mass; carboxylic acid of 0.6-6.0% of total polyacrylate mass; and a combination of amine content of 0.1-1.0% and carboxylic acid of 0.3-3.0% of total polyacrylate mass.

When a microcapsule's shell includes a polyacrylate material, and the shell has an overall mass, the polyacrylate material can form 5-100% of the overall mass, or any integer value for percentage in this range, or any range formed by any of these values for percentage. As examples, the polyacrylate material can form at least 5%, at least 10%, at least 25%, at least 33%, at least 50%, at least 70%, or at least 90% of the overall mass.

Some or all of the microcapsules can have various shell thicknesses. For at least a first group of the provided microcapsules, each microcapsule can have a shell with an overall thickness of 1-300 nanometers, or any integer value for nanometers in this range, or any range formed by any of these values for thickness. As an example, microcapsules can have a shell with an overall thickness of 2-200 nanometers.

The anhydrous composition can include microcapsules wherein, for at least a first group of the microcapsules, the microcapsules encapsulate one or more benefit agents. The benefit agent(s) can include one or more of chromogens, dyes, antibacterial agents, cooling sensates, warming sensates, perfumes, flavorants, sweeteners, oils, pigments, pharmaceuticals, moldicides, herbicides, fertilizers, phase change materials, adhesives, and any other kind of benefit agent known in the art, in any combination. In some examples, the fragrance encapsulated can have a ClogP of less than 4.5 or a ClogP of less than 4. In some examples, the microcapsule may be anionic, cationic, zwitterionic, or have a neutral charge. The benefit agents(s) can be in the form of solids and/or liquids. The benefit agent(s) can be any kind of fragrance(s) known in the art, in any combination.

In some examples, the microcapsule's shell comprises a reaction product of a first mixture in the presence of a second mixture comprising an emulsifier, the first mixture comprising a reaction product of i) an oil soluble or dispersible amine with ii) a multifunctional acrylate or methacrylate monomer or oligomer, an oil soluble acid and an initiator, the emulsifier comprising a water soluble or water dispersible acrylic acid alkyl acid copolymer, an alkali or alkali salt, and optionally a water phase initiator. In some examples, said amine is an aminoalkyl acrylate or aminoalkyl methacrylate.

In some examples, the microcapsules include a core material and a shell surrounding the core material, wherein the shell comprises: a plurality of amine monomers selected from the group consisting of aminoalkyl acrylates, alkyl aminoalkyl acrylates, dialkyl aminoalykl acrylates, aminoalkyl methacrylates, alkylamino aminoalkyl methacrylates, dialkyl aminoalykl methacrylates, tertiarybutyl aminethyl methacrylates, diethylaminoethyl methacrylates, dimethylaminoethyl methacrylates, dipropylaminoethyl methacrylates, and mixtures thereof; and a plurality of multifunctional monomers or multifunctional oligomers.

In some examples, the microcapsule may be spray-dried to form spray-dried microcapsules. Spray-dried microcapsules may be employed in anhydrous compositions. For example, a polyacrylate microcapsule encapsulating a fragrance may be spray-dried before inclusion in an anhydrous composition, the anhydrous composition including a parent fragrance. The cyclodextrin may also be spray-dried before inclusion in the anhydrous composition.

The composition can also contain one or more additional delivery systems for providing one or more benefit agents, in addition to the microcapsules. The additional delivery system(s) can differ in kind from the microcapsules. For example, wherein the microcapsules encapsulates a fragrance, the additional delivery system can be an additional fragrance delivery system, such as a moisture-triggered fragrance delivery system. In the presently claimed invention, the composition further comprises a moisture-triggered fragrance delivery technology comprising a second non-parent fragrance; wherein the second non-parent fragrance is encapsulated in cyclodextrin particles.

Some fragrances can be considered to be volatiles and other fragrances can be considered to be or non-volatiles, as described and defined herein. The term "non-volatile," as used herein, unless otherwise specified, refers to those materials that are liquid under ambient conditions and which have a measurable vapor pressure at 25° C. These materials typically have a vapor pressure less than 0.01 mmHg, and an average boiling point typically greater than 250° C. The term "volatile," as used herein, unless otherwise specified, refers to those materials that are liquid under ambient conditions and which have a measurable vapor pressure at 25° C. These materials typically have a vapor pressure greater than 0.01 mmHg, more typically from 0.02 mmHg to 20 mmHg, and an average boiling point typically less than 250° C, more typically less than 235° C.

A composition or article can comprise a parent fragrance that is dispersed throughout the composition or article, wherein the parent fragrance is made from a parent plurality of fragrance components; and microcapsules, wherein for at least a first group of microcapsules (and optionally, also for a second group of microcapsules), the microcapsules encapsulate a non-parent fragrance,; wherein a first Friction Sample Headspace Ratio Average of the non-parent fragranceto the parent fragrance, when calculated with the Headspace Analysis Test Method, is greater than or equal to 2.8, greater than or equal to 4.2, greater than or equal to 5.6, and/or less than 400.

A composition or article can comprise a parent fragrance that is dispersed throughout the composition or article, wherein the parent fragrance is made from a parent plurality of fragrance components; and microcapsules, wherein for at least a first group of the microcapsules (and optionally, also for a second group of microcapsules), each of the microcapsules encapsulates a first fragrance, which is made from a first plurality of fragrance components; wherein a first Friction Sample Headspace Ratio Maximum of the first plurality of fragrance components to the parent plurality of fragrance components, when calculated with the Headspace Analysis Test Method, is greater than or equal to 10, greater than or equal to 20, greater than or equal to 50, and/or less than 400.

A composition or article can comprise a parent fragrance that is dispersed throughout the composition or article, wherein the parent fragrance is made from a parent plurality of fragrance components; and microcapsules, wherein for at least a first group of the microcapsules (and optionally, also for a second group of microcapsules), each of the microcapsules encapsulates a first fragrance, which is made from a first plurality of fragrance components; wherein a first Moisture Sample Headspace Ratio Average of the first plurality of fragrance components to the parent plurality of fragrance components, when calculated with the Headspace Analysis Test Method, is greater than or equal to 6, greater than or equal to 9, greater than or equal to 12, and/or less than 400.

A composition or article can comprise a parent fragrance that is dispersed throughout the composition or article, wherein the parent fragrance is made from a parent plurality of fragrance components; and microcapsules, wherein for at least a first group of the microcapsules (and optionally, also for a second group of microcapsules), each of the microcapsules encapsulates a first fragrance, which is made from a first plurality of fragrance components; wherein a first Moisture Sample Headspace Ratio Maximum of the first plurality of fragrance components to the parent plurality of fragrance components, when calculated with the Headspace Analysis Test Method, is greater than or equal to 25, greater than or equal to 40, greater than or equal to 100, and/or less than 400.

The composition can be selected from the group including: solid antiperspirant, fluid antiperspirant, and solid deodorant, fluid deodorant,.

The composition includes a first group of microcapsules and a second group of microcapsules, for at least the first group of the microcapsules, the microcapsules is a first kind of microcapsule, configured with a first delivery technology, and for at least the second group of the microcapsules, each of the microcapsules is a second kind of microcapsule, configured with a second delivery technology that differs from the first delivery technology. Non-limited examples of delivery technologies include friction-triggered fragrance technologies (e.g. polyacrylate microcapsules) and moisture-triggered fragrance technologies (e.g. beta-cyclodextrin).

As a result, a composition has a parent fragrance and a moisture-triggered fragrance delivery technology that provide bursts of fragrance when triggered by a threshold level of moisture, providing a noticeable fragrance when the fragrance of the moisture-triggered fragrance delivery technology differs from the parent fragrance; further, if the personal care product also has a composition with a friction-triggered fragrance technology, then the composition can provide additional bursts of fragrance when triggered by friction and can provide additional noticeable fragrance when the fragrance of the friction triggered fragrance delivery technology also differs from the parent fragrance.

The anhydrous composition can be any kind of composition disclosed herein or known in the art. For example, the anhydrous composition can be a composition such as a semi-solid deodorant, semi-solid antiperspirant, an invisible solid deodorant, an invisible solid antiperspirant, aerosol antiperspirant, fluid antiperspirant, body powder, and foot powder.

### Cyclic Oligosaccharides

The compositions or articles described herein include a moisture-triggered fragrance technology incorporating cyclic oligosaccharides -- the second non-parent fragrance is encapsulated in cyclodextrin particles.

As used herein, the term "cyclic oligosaccharide" means a cyclic structure comprising six or more saccharide units. The cyclic oligosaccharides can have six, seven, or eight saccharide units or mixtures thereof. It is common in the art to refer to six, seven and eight membered cyclic oligosaccharides as α, β, and γ, respectively. The cyclic oligosaccharides that may be useful include those that are soluble in water, ethanol, or both water and ethanol. The cyclic oligosaccharides useful herein may have a solubility of at least 0.1g/100 ml, at 25° C and 1 atm of pressure in either water, ethanol, or both water and ethanol. The compositions disclosed herein may comprise from 0.001% to 40%, from 0.1% to 25%, from 0.3% to 20%, from 0.5% to 10%, or from 0.75% to 5%, by weight of the composition, of a cyclic oligosaccharide. The compositions disclosed herein may comprise from 0.001% to 40%, from.1% to 25%, from 0.3% to 20%, from 0.5% to 10%, or from 0.75% to 5%, by weight of the composition, of a cyclic oligosaccharide.

The cyclic oligosaccharide may comprise any suitable saccharide or mixture of saccharides. Examples of suitable saccharides include, but are not limited to, glucose, fructose, mannose, galactose, maltose, and mixtures thereof. The cyclic oligosaccharide, or mixture of cyclic oligosaccharides, may be substituted by any suitable substituent or mixture of substituents. Herein the use of the term "mixture of substituents" means that two or more different suitable substituents may be substituted onto one cyclic oligosaccharide. Suitable examples of substituents include, but are not limited to, alkyl groups, hydroxyalkyl groups, dihydroxyalkyl groups, carboxyalkyl groups, aryl groups, maltosyl groups, allyl groups, benzyl groups, alkanoyl groups, and mixtures thereof. These substituents may be saturated or unsaturated, straight or branched chain. For example, the substituents may include saturated and straight chain alkyl groups, hydroxyalkyl groups, and mixtures thereof. The alkyl and hydroxyalkyl substituents, for example, may also be selected from C₁-C₈ alkyl or hydroxyalkyl groups, alkyl and hydroxyalkyl substituents from C₁-C₆alkyl or hydroxyalkyl groups, and alkyl and hydroxyalkyl substituents from C₁-C₄ alkyl or hydroxyalkyl groups. The alkyl and hydroxyalkyl substituents may be, for example, propyl, ethyl, methyl, and hydroxypropyl.

In addition to the substituents themselves, the cyclic oligosaccharides may have an average degree of substitution of at least 1.6, wherein the term "degree of substitution" means the average number of substituents per saccharide unit. For example, the cyclic oligosaccharides may have an average degree of substitution of less than 2.8 or from 1.7 to 2.0. The average number of substituents may be determined using common Nuclear Magnetic Resonance techniques known in the art. Examples of cyclic oligosaccharides useful herein include cthe cyclodextrins such as methyl-α-cyclodextrins, methyl-β-cyclodextrins, hydroxypropyl-α-cyclodextrins, hydroxypropyl-β-cyclodextrins, and mixtures thereof. In the claimed invention, the second non-parent fragrance is encapsulated in cyclodextrin particles. The cyclodextrins may also be spray-dried and may also be spray-dried particles.

### Fragrances

The compositions or articles comprise fragrances. As used herein, "fragrance" is used to indicate any odoriferous material. Any fragrance that is cosmetically acceptable may be used in the composition. For example, the fragrance may be one that is a liquid at room temperature. Generally, the fragrance(s) may be present at a level from 0.01% to 40%, from 0.1% to 25%, from 0.25% to 20%, or from 0.5% to 15%, by weight of the composition.

A wide variety of chemicals are known as fragrances, including aldehydes, ketones, and esters. More commonly, naturally occurring plant and animal oils and exudates comprising complex mixtures of various chemical components are known for use as fragrances. Non-limiting examples of the fragrances useful herein include pro-fragrances such as acetal pro-fragrances, ketal pro-fragrances, ester pro-fragrances, hydrolyzable inorganic-organic pro-fragrances, and mixtures thereof. The fragrances may be released from the pro-fragrances in a number of ways. For example, the fragrance may be released as a result of simple hydrolysis, or by a shift in an equilibrium reaction, or by a pH-change, or by enzymatic release. The fragrances herein may be relatively simple in their chemical make-up, comprising a single chemical, or may comprise highly sophisticated complex mixtures of natural and synthetic chemical components, all chosen to provide any desired odor.

The fragrances may have a boiling point (BP) of 500°C or lower, 400°C or lower, or 350°C or lower. The BP of many fragrances are disclosed in Perfume and Flavor Chemicals (Aroma Chemicals), Steffen Arctander (1969). The ClogP value of the fragrances may be 0.1 or greater, 0.5 or greater, 1.0 or greater, and 1.2 or greater. As used herein, "ClogP" means the logarithm to the base 10 of the octanol/water partition coefficient. The ClogP can be readily calculated from a program called "CLOGP" which is available from Daylight Chemical Information Systems Inc., Irvine Calif., USA. Octanol/water partition coefficients are described in more detail in U.S. Pat. No. 5,578,563.

Suitable fragrances are also disclosed in U.S. Pat. No. 4,145,184, U.S. Pat. No. 4,209,417, U.S. Pat. No. 4,515,705, and U.S. Pat. No. 4,152,272. Non-limiting examples of fragrances include animal fragrances such as musk oil, civet, castoreum, ambergris, plant fragrances such as nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

Other examples of suitable fragrances include, but are not limited to, chemical substances such as acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenylacetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

### Adjunct Ingredients

The non-limiting list of adjunct ingredients illustrated hereinafter are suitable for use in compositions and/or articles and may be desirably incorporated, for example to assist or enhance performance, for treatment of the substrate to be cleaned, or to modify the aesthetics of the composition as is the case with perfumes, colorants, dyes or the like. It is understood that such adjuncts are in addition to the components that are supplied via the microcapsules. The precise nature of these adjunct ingredients, and levels of incorporation thereof, will depend on the physical form of the composition and the nature of the operation for which it is to be used. Suitable adjunct materials include, but are not limited to, polymers, for example cationic polymers, surfactants, builders, chelating agents, dye transfer inhibiting agents, dispersants, enzymes, enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments, antiperspirant actives, skin care actives (e.g. niacinamide), glycerin, and mixtures thereof. In some examples, the adjunct may be a carrier like water. It is also envisioned that more than one type of adjunct ingredient may be included in the composition.

### Method of Use

The compositions described herein may be packaged with any container known in the art or with any dispenser suitable for delivering the composition to a substrate. The composition may be applied to any substance where moisture and/or friction is available to trigger the release of the fragrance. When the composition is applied to the human body, the composition may be applied to any area of the skin or may be applied to any area of the body. The compositions may be used as consumer products (i.e. products intended to be sold to consumers without further modification or processing). Moreover, the microcapsules may be applied to any article, such as a fabric or any absorbent material including, but not limited to, feminine hygiene products, diapers, and adult incontinence products. The composition may also be incorporated into an article.

The compositions and articles described herein may also be used to overcome the habituation experienced by some consumers to a parent fragrance in an article and/or composition. For example, some consumers are known to suffer from habituation to the fragrance expressed by a composition and/or article such that the fragrance becomes less noticeable over time. One method of overcoming habituation in a composition and/or article is to incorporate a non-parent fragrance and a parent fragrance wherein the fragrances are expressed at different times or in an oscillating fashion. However, this is not easily done in practice as simply mixing the parent fragrance and non-parent fragrance together may result in a bloom that is a combination of both types of fragrances such that some consumers may still experience habituation to the combination. Additionally, many encapsulation technologies that allow for a triggered- or delayed-release of a non-parent fragrance result in some level of mixing of the parent and non-parent fragrances.

In this regard, some encapsulation technologies may not effectively prevent diffusion of the encapsulated, non-parent fragrance into the composition and/or the diffusion of the parent fragrance into the core of the encapsulation material such that there is a mixing of the parent fragrance and the non-parent fragrance. Although such technologies may provide a triggered- or delayed-release of a fragrance, these technologies may not be able to overcome the habituation as a result of the mixing of the parent and non-parent fragrances that occurs before the consumer uses the product.

Thus, the technologies described herein may overcome habituation in a composition and/or article by delaying the release of the non-parent fragrance so that the parent fragrance and the non-parent fragrance(s) bloom at different times and such that the non-parent and parent fragrance do not mix in the composition and/or article to a significant degree before usage. The technologies described herein can also be used to combat habituation without the need for a moisture-triggering event. For example, friction alone may be a sufficient triggering event that is used to express the non-parent fragrance. Additionally, the technologies described herein may also be used to combat habituation by utilizing multiple different classes of triggering events, such as moisture-triggering events and friction-triggered events.

### Solid Antiperspirant Compositions

Anhydrous compositions, like solid antiperspirant compositions, may require microcapsules with less than 20% water, preferably with less than 5% water. Free water in such anhydrous compositions can lead to the crystallization of the antiperspirant actives which may affect the performance of the composition when used. Spray-drying a slurry of microcapsules before inclusion into a solid antiperspirant composition is one way of reducing the amount of water associated with the microcapsules. Other ways of reducing the moisture content of the microcapsules are known, such as drying the microcapsules in an oven.

Additionally, for at least some friable microcapsules, such microcapsules may be more flexible in environments containing high levels of water. For example, for at least some microcapsules, said microcapsules may not release their core material (e.g. a fragrance) when friction or other mechanical forces are applied in a hyper-hydrated state. By spray-drying said microcapsules before inclusion into the anhydrous composition, said microcapsules may be more likely to rupture and release their core materials.

Solid antiperspirant compositions may include an antiperspirant active suitable for application to human skin. The concentration of the antiperspirant active in the composition should be sufficient to provide the desired enhanced wetness protection. For example, the active may be present in an amount of from 0.1%, 0.5%, 1%, 5%, or 10%; to 60%, 35%, 30%, 25% or 20%, by weight of the composition. These weight percentages are calculated on an anhydrous metal salt basis exclusive of water and any complexing agents such as glycine, glycine salts, or other complexing agents.

An antiperspirant active can include any compound, composition, or other material having antiperspirant activity. Such actives may include astringent metallic salts, especially inorganic and organic salts of aluminum, zirconium and zinc, as well as mixtures thereof. For example, the antiperspirant actives may include zirconium-containing salts or materials, such as zirconyl oxyhalides, zirconyl hydroxyhalides, and mixtures thereof; and/or aluminum-containing salts such as, for example, aluminum halides, aluminum chlorohydrate, aluminum hydroxyhalides, and mixtures thereof.

### 1. Aluminum Salts

Aluminum salts useful herein can include those that conform to the formula:

Al₂(OH)ₐCl_{b} · x H₂O

wherein a is from 2 to 5; the sum of a and b is 6; x is from 1 to 6; where a, b, and x may have non-integer values. For example, aluminum chlorohydroxides referred to as "5/6 basic chlorohydroxide," wherein a is 5 and "2/3 basic chlorohydroxide", wherein a=4 may be used.

### 2. Zirconium Salts

Zirconium salts useful herein can include those which conform to the formula:

ZrO(OH)₂₋ₐClₐ · x H₂O

wherein a is from 1.5 to 1.87; x is from 1 to 7; and wherein a and x may both have non-integer values. Useful are zirconium salt complexes that additionally contain aluminum and glycine, commonly known as "ZAG complexes". These complexes can contain aluminum chlorohydroxide and zirconyl hydroxy chloride conforming to the above-described formulas. Examples of two such complexes include aluminum zirconium trichlorohydrex and aluminum zirconium tetrachlorohydrex.

Antiperspirant compositions can also include a structurant to help provide the composition with the desired viscosity, rheology, texture and/or product hardness, or to otherwise help suspend any dispersed solids or liquids within the composition. The term "structurant" may include any material known or otherwise effective in providing suspending, gelling, viscosifying, solidifying, or thickening properties to the composition or which otherwise provide structure to the final product form. These structurants may include, for example, gelling agents, polymeric or nonpolymeric agents, inorganic thickening agents, or viscosifying agents. The thickening agents may include, for example, organic solids, silicone solids, crystalline or other gellants, inorganic particulates such as clays or silicas, or combinations thereof.

The concentration and type of the structurant selected for use in the antiperspirant composition will vary depending upon the desired product form, viscosity, and hardness. The structurant suitable for use herein, may have a concentration range from 0.1%, 2%, 3%, 5%; or 10%; to 35%, 20%, 10%, or 8%, by weight of the composition. Soft solids will often contain a lower amount of structurant than solid compositions. For example, a soft solid may contain from 1.0% to 9%, by weight of the composition, while a solid composition may contain from 15% to 25%, by weight of the composition, of structurant. This is not a hard and fast rule, however, as a soft solid product with a higher structurant value can be formed by, for example, shearing the product as it is dispensed from a package.

Non-limiting examples of suitable gelling agents include fatty acid gellants, salts of fatty acids, hydroxyl acids, hydroxyl acid gellants, esters and amides of fatty acid or hydroxyl fatty acid gellants, cholesterolic materials, dibenzylidene alditols, lanolinolic materials, fatty alcohols, triglycerides, sucrose esters such as SEFA behenate, inorganic materials such as clays or silicas, other amide or polyamide gellants, and mixtures thereof.

Suitable gelling agents include fatty acid gellants such as fatty acid and hydroxyl or alpha hydroxyl fatty acids, having from 10 to 40 carbon atoms, and ester and amides of such gelling agents. Non-limiting examples of such gelling agents include, but are not limited to, 12-hydroxystearic acid, 12-hydroxylauric acid, 16-hydroxyhexadecanoic acid, behenic acid, eurcic acid, stearic acid, caprylic acid, lauric acid, isostearic acid, and combinations thereof. Preferred gelling agents are 12-hydroxystearic acid, esters of 12-hydroxystearic acid, amides of 12-hydroxystearic acid and combinations thereof.

Other suitable gelling agents include amide gellants such as di-substituted or branched monoamide gellants, monsubstituted or branched diamide gellants, triamide gellants, and combinations thereof, including n-acyl amino acid derivatives such as n-acyl amino acid amides, n-acyl amino acid esters prepared from glutamic acid, lysine, glutamine, aspartic acid, and combinations thereof.

Still other examples of suitable gelling agents include fatty alcohols having at least 8 carbon atoms, at least 12 carbon atoms but no more than 40 carbon atoms, no more than 30 carbon atoms, or no more than 18 carbon atoms. For example, fatty alcohols include but are not limited to cetyl alcohol, myristyl alcohol, stearyl alcohol and combinations thereof.

Non-limiting examples of suitable tryiglyceride gellants include tristearin, hydrogenated vegetable oil, trihydroxysterin (Thixcin® R, available from Rheox, Inc.), rape seed oil, castor wax, fish oils, tripalmitin, Syncrowax® HRC and Syncrowax® HGL-C (Syncrowax® available from Croda, Inc.).

Other suitable structurants include waxes or wax-like materials having a melt point of above 65°C, more typically from 65°C to 130°C, examples of which include, but are not limited to, waxes such as beeswax, carnauba, bayberry, candelilla, montan, ozokerite, ceresin, hydrogenated castor oil (castor wax), synthetic waxes and microcrystalline waxes. Castor wax is preferred within this group. The synthetic wax may be, for example, a polyethylene, a polymethylene, or a combination thereof. Some suitable polymethylenes may have a melting point from 65°C to 75°C. Examples of suitable polyethylenes include those with a melting point from 60°C to 95°C.

Further structurants for use in the solid antiperspirant compositions of the present invention may include inorganic particulate thickening agents such as clays and colloidal pyrogenic silica pigments. For example, colloidal pyrogenic silica pigments such as Cab-O-Sil®, a submicroscopic particulated pyrogenic silica may be used. Other known or otherwise effective inorganic particulate thickening agents that are commonly used in the art can also be used in the solid antiperspirant compositions of the present invention. Concentrations of particulate thickening agents may range, for example, from 0.1%, 1%, or 5%; to 35%, 15%, 10% or 8%, by weight of the composition.

Suitable clay structurants include montmorillonite clays, examples of which include bentonites, hectorites, and colloidal magnesium aluminum silicates. These and other suitable clays may be hydrophobically treated, and when so treated will generally be used in combination with a clay activator. Non-limiting examples of suitable clay activators include propylene carbonate, ethanol, and combinations thereof. When clay activators are present, the amount of clay activator will typically range from 40%, 25%, or 15%; to 75%, 60%, or 50%, by weight of the clay.

Solid antiperspirant compositions may further include anhydrous liquid carriers. These are present, for example, at concentrations ranging from 10%, 15%, 20%, 25%; to 99%, 70%, 60%, or 50%, by weight of the composition. Such concentrations will vary depending upon variables such as product form, desired product hardness, and selection of other ingredients in the composition. The anhydrous carrier may be any anhydrous carrier known for use in personal care applications or otherwise suitable for topical application to the skin. For example, anhydrous carriers may include, but are not limited to volatile and nonvolatile fluids.

An antiperspirant composition may further include a volatile fluid such as a volatile silicone carrier. Volatile fluids are present, for example, at concentrations ranging from 20% or from 30%; to 80%, or no 60%, by weight of the composition. The volatile silicone of the solvent may be cyclic, linear, and/or branched chain silicone. "Volatile silicone", as used herein, refers to those silicone materials that have measurable vapor pressure under ambient conditions.

The volatile silicone may be a cyclic silicone. The cyclic silicone may have from 3 silicone atoms, or from 5 silicone atoms; to 7 silicone atoms, or 6 silicone atoms. For example, volatile silicones may be used which conform to the formula: 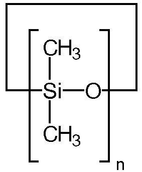 wherein n is from 3, or from 5; to 7, or 6. These volatile cyclic silicones generally have a viscosity of less than 10 mPa.s (10 centistokes) at 25 °C. Suitable volatile silicones for use herein include, but are not limited to, Cyclomethicone D5 (commercially available from G. E. Silicones); Dow Corning 344, and Dow Corning 345 (commercially available from Dow Corning Corp.); and GE 7207, GE 7158 and Silicone Fluids SF-1202 and SF-1173 (available from General Electric Co.). SWS-03314, SWS-03400, F-222, F-223, F-250, F-251 (available from SWS Silicones Corp.); Volatile Silicones 7158, 7207, 7349 (available from Union Carbide); Masil SF-V (available from Mazer) and combinations thereof.

An antiperspirant composition may further comprise a non-volatile fluid. These non-volatile fluids may be either non-volatile organic fluids or non-volatile silicone fluids. The non-volatile organic fluid can be present, for example, at concentrations ranging from 1%, from 2%; to 20%, or 15%, by weight of the composition.

Non-limiting examples of nonvolatile organic fluids include, but are not limited to, mineral oil, PPG-14 butyl ether, isopropyl myristate, petrolatum, butyl stearate, cetyl octanoate, butyl myristate, myristyl myristate, C12-15 alkylbenzoate (e.g., Finsolv.TM.), dipropylene glycol dibenzoate, PPG-15 stearyl ether benzoate and blends thereof (e.g. Finsolv TPP), neopentyl glycol diheptanoate (e.g. Lexfeel 7 supplied by Inolex), octyldodecanol, isostearyl isostearate, octododecyl benzoate, isostearyl lactate, isostearyl palmitate, isononyl/ isononoate, isoeicosane, octyldodecyl neopentanate, hydrogenated polyisobutane, and isobutyl stearate.

An antiperspirant composition may further include a non-volatile silicone fluid. The non-volatile silicone fluid may be a liquid at or below human skin temperature, or otherwise in liquid form within the anhydrous antiperspirant composition during or shortly after topical application. The concentration of the non-volatile silicone may be from 1%, from 2%; to 15%, 10%, by weight of the composition. Nonvolatile silicone fluids of the present invention may include those which conform to the formula: 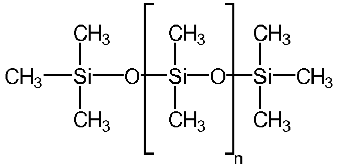 wherein n is greater than or equal to 1. These linear silicone materials may generally have viscosity values of from 5 mPa.s (5 centistokes), from 10 mPa.s (10 centistokes); to 100,000 mPa.s (100,000 centistokes), 500 mPa.s (500 centistokes), 200 mPa.s (200 centistokes), or 50 mPa.s (50 centistokes), as measured under ambient conditions.

Specific non limiting examples of suitable nonvolatile silicone fluids include Dow Corning 200, hexamethyldisiloxane, Dow Corning 225, Dow Corning 1732, Dow Coming 5732, Dow Coming 5750 (available from Dow Corning Corp.); and SF-96, SF-1066 and SF18(350) Silicone Fluids (available from G.E. Silicones).

Low surface tension non-volatile solvent may be also be used. Such solvents may be selected from the group consisting of dimethicones, dimethicone copolyols, phenyl trimethicones, alkyl dimethicones, alkyl methicones, and mixtures thereof. Low surface tension non-volatile solvents are also described in U.S. Pat. No. 6,835,373 (Kolodzik et al.).

An antiperspirant composition may include a malodor reducing agent. Malodor reducing agents include components other than the antiperspirant active within the composition that act to eliminate the effect that body odor has on fragrance display. These agents may combine with the offensive body odor so that they are not detectable including, but not limited to, suppressing evaporation of malodor from the body, absorbing sweat or malodor, masking the malodor or microbiological activity on odor causing organisms. The concentration of the malodor reducing agent within the composition is sufficient to provide such chemical or biological means for reducing or eliminating body odor. Although the concentration will vary depending on the agent used, generally, the malodor reducing agent may be included within the composition from 0.05%, 0.5%, or 1%; to 15%, 10%, or 6%, by weight of the composition.

Malodor reducing agents may include, but are not limited to, pantothenic acid and its derivatives, petrolatum, menthyl acetate, uncomplexed cyclodextrins and derivatives thereof, talc, silica and mixtures thereof.

For example, if panthenyl triacetate is used, the concentration of the malodor reducing agent may be from 0.1% or 0.25%; to 3.0%, or 2.0%, by weight of the composition. Another example of a malodor reducing agent is petrolatum which may be included from 0.10%, or 0.5%; to 15%, or 10%, by weight of the composition. A combination may also be used as the malodor reducing agent including, but not limited to, panthenyl triacetate and petrolatum at levels from 0.1%, or 0.5%; to 3.0 %, or 10%, by weight of the composition. Menthyl acetate, a derivative of menthol that does not have a cooling effect, may be included from 0.05%, or 0.01%; to 2.0%, or 1.0%, by weight of the composition. The malodor reducing agent may be in the form of a liquid or a semi-solid such that it does not contribute to product residue.

### Examples

### First Comparative Example

The following, in Table 1A, is data related to a first Comparative Example of an antiperspirant product known in the prior art, wherein the product includes a composition known to have a parent fragrance, no friction-triggered fragrance delivery technology, and no moisture triggered fragrance delivery technology. The first Comparative Example was subjected to the Headspace Analysis Test Method, which generated the four chromatograms in Figure 1, with chart 101 indicating First Headspace profile, chart 102 indicating Second Headspace profile, chart 103 indicating Third Headspace profile, and chart 104 indicating Fourth Headspace profile. Table 1A shows the components of the antiperspirant product, along with results from the First, Second, Third, and Fourth Headspace Values, each of which was calculated according to the Headspace Analysis Test Method described herein.

**Table 1A**

| Component | First Headspace Value | Second Headspace Value | Third Headspace Value | Fourth Headspace Value |
|---|---|---|---|---|
| Hexanal | 1 | 2.5 | 2.2 | 12.3 |
| ethyl-2-methylbutyrate | 1 | 0 | 0 | 0 |
| 3-methyl, 2-butenol acetate | 1 | 0 | 1.2 | 0 |
| Tricyclene | 1 | 0 | 0 | 0 |
| 6-me-5-hepten-2-one | 1 | 1.9 | 2.3 | 4.8 |
| beta-pinene | 1 | 0 | 0 | 0 |
| cis-3-hexenyl acetate | 1 | 0 | 0.1 | 0.1 |
| hexyl ester, acetic acid | 1 | 0 | 0 | 0 |
| d-limonene | 1 | 0 | 0 | 0 |
| dihydro myrcenol | 1 | 0 | 0 | 0 |
| acetyl caproyl | 1 | 0 | 0 | 0 |
| Linalool | 1 | 0 | 0 | 0 |
| Nonanal | 1 | 0.8 | 1.1 | 2 |
| benzyl acetate | 1 | 0 | 0 | 0.2 |
| allyl heptanoate | 1 | 0 | 0 | 0 |
| ethyl linalool isomer 1 | 1 | 0 | 0 | 0.3 |
| ethyl linalool isomer 2 | 1 | 0 | 0.1 | 0.4 |
| Florol Major 1 | 1 | 0.4 | 0.7 | 1.1 |
| Decanal | 1 | 1.7 | 2.5 | 4.3 |
| Florol Major 2 | 1 | 1 | 1.5 | 2 |
| thesaron major | 1 | 0 | 0 | 0.1 |
| verdox major | 1 | 0.1 | 0.1 | 0.3 |
| beta ionone | 1 | 1.6 | 2.9 | 5.4 |
| dimethyl benz carb butyrate | 1 | 2.6 | 4.6 | 7.7 |
| Lilial | 1 | 1.6 | 2.5 | 4.6 |
| hexyl salicylate | 1 | 3.5 | 5.3 | 7.8 |
| benzyl benzoate | 1 | 2.5 | 3.5 | 4.4 |
| Galaxolide | 1 | 2.2 | 2.9 | 3.8 |
| | | | | |
| AVERAGE | | | 1.2 | 2.2 |
| MAXIMUM | | | 5.3 | 12.3 |

As shown in Table 1A, and as calculated according to the Headspace Analysis Test Method described herein for the first Comparative Example: the Friction Sample Headspace Ratio Average is 1.2, and the Friction Sample Headspace Ratio Maximum is 5.3. These relatively small ratio values accurately indicate the absence of a friction-triggered perfume delivery technology in the product of the first Comparative Example.

As shown in Table 1A, and as calculated according to the Headspace Analysis Test Method described herein for the first Comparative Example: the Moisture Sample Headspace Ratio Average is 2.2, and the Moisture Sample Headspace Ratio Maximum is 12.3. These relatively small ratio values accurately indicate the absence of a moisture-triggered perfume delivery technology in the product of the first Comparative Example.

### Second Comparative Example

The following, in Table 2A, is data related to a second Comparative Example of a deodorant/antiperspirant product known in the prior art, wherein the product includes a composition known to have a parent fragrance and a moisture-triggered fragrance delivery technology (beta-cyclodextrin) for a fragrance that differs from the parent fragrance. The second Comparative Example was subjected to the Headspace Analysis Test Method, which generated the four chromatograms in Figure 2, with chart 201 indicating First Headspace profile, chart 202 indicating Second Headspace profile, chart 203 indicating Third Headspace profile, and chart 204 indicating Fourth Headspace profile. Table 2A shows the components of the antiperspirant product, along with results from the First, Second, Third, and Fourth Headspace Values, each of which was calculated according to the Headspace Analysis Test Method described herein.

**Table 2A**

| Component | First Headspace Value | Second Headspace Value | Third Headspace Value | Fourth Headspace Value |
|---|---|---|---|---|
| Hexanal | 1 | 2.4 | 1.5 | 7.9 |
| ethyl-2-methylbutyrate | 1 | 0.0 | 0.5 | 404.8 |
| Tricyclene | 1 | 0.0 | 0.0 | 29.7 |
| 6-me-5-hepten-2-one | 1 | 2.5 | 2.7 | 4.8 |
| beta-pinene | 1 | 0.0 | 0.0 | 6.9 |
| cis-3-hexenyl acetate | 1 | 0.0 | 0.1 | 29.3 |
| hexyl ester, acetic acid | 1 | 0.0 | 0.0 | 0.0 |
| d-limonene | 1 | 0.0 | 0.0 | 0.0 |
| dihydro myrcenol | 1 | 0.0 | 0.0 | 0.0 |
| acetyl caproyl | 1 | 0.0 | 0.0 | 0.0 |
| Linalool | 1 | 0.0 | 0.0 | 0.0 |
| Nonanal | 1 | 1.3 | 1.8 | 2.3 |
| benzyl acetate | 1 | 0.0 | 0.0 | 0.1 |
| allyl heptanoate | 1 | 0.0 | 0.0 | 127.5 |
| ethyl linalool isomer 1 | 1 | 0.0 | 0.0 | 2.2 |
| ethyl linalool isomer 2 | 1 | 0.0 | 0.1 | 2.6 |
| Florol Major 1 | 1 | 0.2 | 0.4 | 2.0 |
| Decanal | 1 | 2.0 | 2.9 | 3.4 |
| Florol Major 2 | 1 | 0.3 | 0.6 | 1.0 |
| thesaron major | 1 | 0.0 | 0.0 | 0.0 |
| verdox major | 1 | 0.0 | 0.1 | 9.8 |
| Cymal | 1 | 1.9 | 3.2 | 4.3 |
| beta ionone | 1 | 0.8 | 2.5 | 13.3 |
| dimethyl benz carb butyrate | 1 | 1.4 | 2.3 | 3.5 |
| Lilial | 1 | 1.3 | 2.2 | 3.0 |
| hexyl salicylate | 1 | 3.3 | 5.0 | 7.6 |
| benzyl benzoate | 1 | 1.7 | 2.5 | 3.3 |
| Galaxolide | 1 | 2.1 | 3.1 | 5.5 |
| | | | | |
| AVERAGE | | | 1.1 | 24.1 |
| MAXIMUM | | | 5.0 | 404.8 |

As shown in Table 2A, and as calculated according to the Headspace Analysis Test Method described herein for the second Comparative Example: the Friction Sample Headspace Ratio Average is 1.1, and the Friction Sample Headspace Ratio Maximum is 5.0. These somewhat larger ratio values accurately indicate the absence of a friction-triggered perfume delivery technology in the product of the second Comparative Example.

As shown in Table 2A, and as calculated according to the Headspace Analysis Test Method described herein for the second Comparative Example: the Moisture Sample Headspace Ratio Average is 24.1, and the Moisture Sample Headspace Ratio Maximum is 404.8. These very large ratio values accurately indicate the presence of a moisture-triggered perfume delivery technology (i.e. beta-cyclodextrin) in the product of the second Comparative Example.

### Third Comparative Example

The following, in Table 3A, is data related to a third Comparative Example of a deodorant/antiperspirant product known in the prior art (i.e. Degree MOTIONSENSE™ antiperspirant, with "Fresh Energy" fragrance, available in consumer markets and purchased in 2012), wherein the product includes a composition known to have a parent fragrance, a friction-triggered fragrance delivery technology (e.g. gelatin microcapsules), and a moisture-triggered fragrance delivery technology (starch encapsulated accord). The third Comparative Example was subjected to the Headspace Analysis Test Method, which generated the four chromatograms in Figure 3, with chart 301 indicating First Headspace profile, chart 302 indicating Second Headspace profile, chart 303 indicating Third Headspace profile, and chart 304 indicating Fourth Headspace profile. Table 3A shows the components of the deodorant/antiperspirant product, along with results from the First, Second, Third, and Fourth Headspace Values, each of which was calculated according to the Headspace Analysis Test Method described herein.

**Table 3A**

| Component | First Headspace Value | Second Headspace Value | Third Headspace Value | Fourth Headspace Value |
|---|---|---|---|---|
| Hexanal | 1 | 1.9 | 1.4 | 6.3 |
| alpha pinene | 1 | 0 | 2.6 | 1.7 |
| Camphene | 1 | 0 | 3.1 | 5.4 |
| Benzaldehyde | 1 | 0.6 | 1 | 4.4 |
| 5-methyl-5-heptenone | 1 | 3.2 | 4.4 | 6.6 |
| Myrcene | 1 | 0 | 0.3 | 0.2 |
| cis-3-hexenyl acetate | 1 | 0 | 0.2 | 1 |
| Octanal | 1 | 2.7 | 3.1 | 6.6 |
| acetic acid, hexyl ester | 1 | 0 | 0.1 | 0.5 |
| para cresyl methyl ether | 1 | 0 | 1.8 | 7.7 |
| para cymene | 1 | 0 | 3.1 | 6.1 |
| d-limonene | 1 | 0 | 0.4 | 0.5 |
| Eucalyptol | 1 | 0 | 1.5 | 6.5 |
| gamma terpinene | 1 | 0 | 1.3 | 2 |
| dihydro myrcenol | 1 | 0 | 0.1 | 0.4 |
| ligustral/triplal | 1 | 0 | 1.2 | 6.1 |
| tetrahydro linalool | 1 | 0 | 0.1 | 0.4 |
| Nonanal | 1 | 1.5 | 1.7 | 3.2 |
| benzyl acetate | 1 | 0 | 0.1 | 0.8 |
| me phe car acetate | 1 | 0 | 0 | 0.2 |
| Decanal | 1 | 5.1 | 6.7 | 9.7 |
| linalyl acetate | 1 | 0 | 0.9 | 3.6 |
| anisic aldehyde | 1 | 0.3 | 1.4 | 8.2 |
| verdox major | 1 | 0.1 | 0.2 | 0.6 |
| Heliotropin | 1 | 0.6 | 1.9 | 8.1 |
| methyl cinnamate | 1 | 1 | 2.3 | 7.9 |
| geranyl acetone | 1 | 6.5 | 6.8 | 8.3 |
| gamma methyl ionone | 1 | 2 | 3.8 | 7.7 |
| dimethyl benz car butyrate | 1 | 2.2 | 3.7 | 6.9 |
| phenoxy ethyl iso-butyrate | 1 | 2.3 | 4.1 | 6.6 |
| alpha methyl ionone | 1 | 4.7 | 9.3 | 18.1 |
| methyl dihydrojasmonate | 1 | 0.7 | 1.3 | 2.5 |
| | | | | |
| AVERAGE | | | 2.2 | 4.8 |
| MAXIMUM | | | 9.3 | 18.1 |

As shown in Table 3A, and as calculated according to the Headspace Analysis Test Method described herein for the third Comparative Example: the Friction Sample Headspace Ratio Average is 2.2, and the Friction Sample Headspace Ratio Maximum is 9.3. These somewhat larger ratio values accurately indicate the presence of a friction-triggered perfume delivery technology (e.g. the gelatin microcapsules) in the product of the third Comparative Example.

As shown in Table 3A, and as calculated according to the Headspace Analysis Test Method described herein for the third Comparative Example: the Moisture Sample Headspace Ratio Average is 4.8, and the Moisture Sample Headspace Ratio Maximum is 18.1. These somewhat larger ratio values accurately indicate the presence of a moisture triggered perfume delivery technology (i.e. starch encapsulated accord) in the product of the third Comparative Example.

### Inventive Example

Following, in Table 4A, is data related to a fourth Example of a deodorant/antiperspirant, wherein the product includes a composition with a parent fragrance, a friction-triggered fragrance delivery technology (polyacrylate microcapsules), and a moisture-triggered fragrance delivery technology (beta-cyclodextrin). The fourth Example was subjected to the Headspace Analysis Test Method, which generated the four chromatograms in Figure 4, with chart 401 indicating First Headspace profile, chart 402 indicating Second Headspace profile, chart 403 indicating Third Headspace profile, and chart 404 indicating Fourth Headspace profile. Table 4A shows the components of the deodorant/antiperspirant product, along with results from the First, Second, Third, and Fourth Headspace Values, each of which was calculated according to the Headspace Analysis Test Method described herein.

**Table 4A**

| Component | First Headspace Value | Second Headspace Value | Third Headspace Value | Fourth Headspace Value |
|---|---|---|---|---|
| Hexanal | 1 | 1.8 | 1.6 | 8.1 |
| ethyl-2-methylbutyrate | 1 | 0.1 | 52.6 | 103.1 |
| iso-amyl acetate | 0 | 0.0 | 0.0 | UC. |
| 3-methyl, 2-butenol acetate | 1 | 0.0 | 20.4 | 6.0 |
| 2-me, ethyl ester pentanoic acid | 1 | 0.0 | 24.3 | 8.4 |
| Tricyclene | 1 | 0.0 | 17.1 | 49.2 |
| 6-me-5-hepten-2-one | 1 | 1.8 | 3.8 | 4.0 |
| beta-pinene | 1 | 0.0 | 4.8 | 9.1 |
| ethyl hexanoate | 1 | 0.0 | 9.1 | 4.6 |
| cis-3-hexenyl acetate | 1 | 0.0 | 3.2 | 13.8 |
| hexyl ester, acetic acid | 1 | 0.0 | 5.0 | 3.2 |
| d-limonene | 1 | 0.0 | 0.0 | 0.0 |
| dihydro myrcenol | 1 | 0.0 | 0.3 | 0.2 |
| acetyl caproyl | 1 | 0.0 | 2.3 | 2.1 |
| ethyl heptanoate | 1 | 0.0 | 2.8 | 2.6 |
| Linalool | 1 | 0.0 | 0.3 | 0.2 |
| Nonanal | 1 | 1.2 | 1.5 | 2.5 |
| cis-hexenyl iso-butyrate | 1 | 0.0 | 2.6 | 3.1 |
| benzyl acetate | 1 | 0.0 | 0.0 | 0.1 |
| menthone major | 0 | 0.0 | 0.0 | UC. |
| allyl heptanoate | 1 | 0.0 | 1.7 | 8.3 |
| ethyl linalool isomer 1 | 1 | 0.0 | 0.0 | 2.3 |
| ethyl linalool isomer 2 | 1 | 0.0 | 0.0 | 2.6 |
| Florol Major 1 | 1 | 0.2 | 0.3 | 2.2 |
| Decanal | 1 | 2.2 | 3.1 | 4.7 |
| Florol Major 2 | 1 | 0.6 | 1.0 | 2.3 |
| thesaron major | 1 | 0.0 | 0.5 | 0.7 |
| verdox major | 1 | 0.0 | 1.4 | 2.9 |
| Cymal | 1 | 1.2 | 3.5 | 4.6 |
| beta ionone | 1 | 1.7 | 4.3 | 9.9 |
| dimethyl benz carb butyrate | 1 | 1.2 | 3.4 | 5.1 |
| Lilial | 1 | 1.8 | 3.4 | 4.0 |
| gamma undecalactone | 1 | 2.1 | 4.3 | 6.6 |
| hexyl salicylate | 1 | 1.9 | 5.6 | 7.9 |
| hexyl cinnamic aldehyde | | | | |
| benzyl benzoate | 1 | 2.1 | 3.5 | 4.7 |
| Galaxolide | 1 | 2.3 | 3.8 | 4.8 |
| | | | | |
| AVERAGE | | | 5.6 | 8.6 |
| MAXIMUM | | | 52.6 | 103.1 |

As shown in Table 4A, and as calculated according to the Headspace Analysis Test Method described herein for the fourth Example: the Friction Sample Headspace Ratio Average is 5.6, and the Friction Sample Headspace Ratio Maximum is 52.6. These larger ratio values accurately indicate the presence of a friction-triggered perfume delivery technology (e.g. the polyacrylate microcapsules) in the product of the fourth Example.

As shown in Table 4A, and as calculated according to the Headspace Analysis Test Method described herein for the fourth Example: the Moisture Sample Headspace Ratio Average is 8.6, and the Moisture Sample Headspace Ratio Maximum is 103.1. These larger ratio values accurately indicate the presence of a moisture triggered perfume delivery technology (e.g. beta-cyclodextrin) in the product of the fourth Example.

### Headspace Analysis Test Method

### Apparatus

The following equipment is used for the Headspace Analysis Test Method.
1. A 125 mL glass jar with a cap, which meets or exceeds all analyte specifications of the latest U.S. EPA "Specifications and Guidance for Contaminant-Free Sample Containers." (such as the I-Chem 300 series type bottle, available from Thermo-Fisher-Scientific Inc., Waltham, Massachusetts, USA), having a stir bar attached (e.g. with tape) to the outside surface of the cap. The attached stir bar allows a GERSTEL-Twister bar to be suspended (via magnetic force) in the headspace above the test sample, for headspace sample collection using stir bar sorptive extraction (SBSE).
2. Several clean, conditioned 2 cm GERSTEL-Twister bars (conditioned using Gerstel Tube Conditioner TC-2, heated to 275°C for 10 minutes under 20ml/min helium flow) having a coating thickness of 1.00 mm (stir bar coated with polydimethylsiloxane) supplied by the Gerstel GmbH & Co. KG of Mülheim an der Ruhr, Germany.
3. A laboratory timer.
4. A Gerstel MPS2-TDU/CIS-4 inlet Thermal Desorption Unit.
5. A gas chromatograph, such as an Agilent model 6890, from Agilent Technologies, Inc., Wilmington, DE, United States (or equivalent).
6. A gas chromatography column, such as an Agilent DB-5MS (from Agilent Technologies, Inc.), that is 30 m x 0.250 mm inner diameter, with a film thickness of 1.0 µm (or equivalent).
7. A supply of helium (carrier gas).
8. A gas chromatography detector, such as model Agilent 5973 Mass Selective Detector (from Agilent Technologies, Inc.) having a source temperature of about 230 °C, and an MS Quad temperature of about 150 °C (or equivalent).
9. A personal computer with gas chromatography software (such as Chemstation from Agilent Technologies, Inc.) that includes the ability to identify fragrance components (for example, using mass spectrometry libraries from John Wiley & Sons and the National Institute of Standards and Technology (NIST)) and to integrate detected peaks and to graphically display their presence on a gas chromatography chart.

### Sampling

For sampling in the Headspace Analysis Test Method, a blotter card is created for each test product. A mass of 0.2 grams of the test product is spread evenly on one side of a perfume blotter card approximately 7.6 cm x 12.7 cm in size. Suitable cards included the Professional Aerosol Testing cardboard blotter cards, as supplied by Orlandi Inc. (Farmingdale, New York, USA). The creation of the blotter card marks time equals zero for this method.

Each time that headspace is collected in this method it is collected from the blotter card, for five minutes, using a Twister bar, while the card is inside of a closed 125 mL glass jar. Four different headspace samples are collected, as described below, with the card remaining inside the glass jar at all times except where specified.

First, a first headspace sample is collected, with no stimulus, immediately after the creation of the blotter card. The collection of the first headspace sample is intended to provide a sample for assessing the presence and intensity of any parent fragrance in the composition, upon application.

Second, a second headspace sample is collected, with no stimulus, at time equals five hours. The collection of the second headspace sample is intended to provide a sample for assessing the presence and intensity of any parent fragrance, after a period of time.

Third, a third headspace sample is collected, with a stimulus of rubbing, immediately after the second sample is collected. The blotter card is removed from the jar and folded in half (with the side treated with test product, forming the inside), the outside of the card is firmly rubbed with pressure from a thumb tip using 8 strokes, each stroke being one passage in a single direction across the full width of the card, ensuring that the entire area of the card is affected, then the blotter card is returned to the jar, and the third sample is collected. The collection of the third headspace sample is intended to provide a sample for assessing the presence and intensity of any friction triggered perfume delivery technologies, such as friable microcapsules.

Fourth, a fourth headspace sample is collected, with a stimulus of moisture, immediately after the third sample is collected. The blotter card is removed from the jar and, while fully opened, a fine mist of distilled water is sprayed on the side treated with test product, then the blotter card is returned to the jar, and the fourth sample is collected. The collection of the fourth headspace sample is intended to provide a sample for assessing the presence and intensity of any moisture triggered perfume delivery technologies, such as starch based microcapsules.

### Analysis

After sampling, the Twister bar is transferred to the Gerstel Thermal Desorption Unit. The collected headspace sample is thermally desorbed using the automated Gerstel TDU before cryofocusing and gas chromatography mass spectrometry analysis. The sample is transferred to the proper sample tray in the unit, then loaded and analyzed. A cryogenic trap is cooled to -80°C and helium (flowing at a rate of about 50 ml/min) is used to purge the trap. The desorption temperature is ramped from 30°C to 265°C and the tube is purged for 3 minutes. The cryo-trap is then heated to remove the trapped fragrances (up to 275°C and held for 3 minutes), followed by the start of the gas chromatography mass spectrometry analysis (run in splitless mode). The following temperature program is used: an initial temperature of about 40 °C which is held for 1 minute, and an increase in the initial temperature at a rate of about 8 °C/min until a temperature of about 250 °C is reached, then an increase of 20 °C/min to 275 °C, to be held for about 3 minutes. The gas chromatography software uses mass spectrometry libraries to identify the components of the fragrances in the collected headspace sample, to integrate detected peaks, and to graphically display their presence on a gas chromatography chart.

### Calculating

For each fragrance ingredient identified in the analyzing of a test product, the intensity value for each ingredient is normalized to the intensity of the first headspace sample - that is, the absolute value of each headspace sample is divided by the absolute value of the first headspace sample, such that the first headspace value is represented as a 1, and each of the second, third, and fourth headspace values is represented by a unitless number that is a multiple of the first headspace value. So, for each component, each of the second, third, and fourth headspace values is a ratio of that headspace value to the first headspace value.

Since the third headspace value is collected with the stimulus of rubbing, the Friction Sample Headspace Ratio Average for a product is calculated as the sum of all of third headspace values for that product, divided by the number of components. The Friction Sample Headspace Ratio Maximum is determined to be the largest value among all of the third headspace values, for that product.

Since the fourth headspace value is collected with the stimulus of moisture, the Moisture Sample Headspace Ratio Average for a product is calculated as the sum of all of fourth headspace values for that product, divided by the number of components. The Moisture Sample Headspace Ratio Maximum is determined to be the largest value among all of the fourth headspace values, for that product.

### Fracture Strength Test Method

One skilled in the art will recognize that various protocols may be constructed for the extraction and isolation of microcapsules from finished products, and will recognize that such methods require validation via a comparison of the resulting measured values, as measured before and after the microcapsules' addition to and extraction from the finished product. The isolated microcapsules are then formulated in de-ionized (DI) water to form a slurry for characterization.

To calculate the percentage of microcapsules which fall within a claimed range of fracture strengths, three different measurements are made and two resulting graphs are utilized. The three separate measurements are namely: i) the volume-weighted particle size distribution (PSD) of the microcapsules; ii) the diameter of at least 10 individual microcapsules within each of 3 specified size ranges, and; iii) the rupture-force of those same 30 or more individual microcapsules. The two graphs created are namely: a plot of the volume-weighted particle size distribution data collected at i) above; and a plot of the modeled distribution of the relationship between microcapsule diameter and fracture-strength, derived from the data collected at ii) and iii) above. The modeled relationship plot enables the microcapsules within a claimed strength range to be identified as a specific region under the volume-weighted PSD curve, and then calculated as a percentage of the total area under the curve.
a.) The volume-weighted particle size distribution (PSD) of the microcapsules is determined via single-particle optical sensing (SPOS), also called optical particle counting (OPC), using the AccuSizer 780 AD instrument, or equivalent, and the accompanying software CW788 version 1.82 (Particle Sizing Systems, Santa Barbara, California, U.S.A.) . The instrument is configured with the following conditions and selections: Flow Rate = 1 ml / sec; Lower Size Threshold = 0.50 µm; Sensor Model Number = LE400-05SE; Autodilution = On; Collection time = 120 sec; Number channels = 512; Vessel fluid volume = 50ml; Max coincidence = 9200 . The measurement is initiated by putting the sensor into a cold state by flushing with water until background counts are less than 100. A capsule slurry, and its density of particles is adjusted with DI water as necessary via autodilution to result in particle counts of at least 9200 per ml. During a time period of 120 seconds the suspension is analyzed. The resulting volume-weighted PSD data are plotted and recorded, and the values of the mean, 5^{th} percentile, and 90^{th} percentile are determined.
b.) The diameter and the rupture-force value (also known as the bursting-force value) of individual microcapsules are measured via a computer-controlled micromanipulation instrument system which possesses lenses and cameras able to image the microcapsules, and which possesses a fine, flat-ended probe connected to a force-transducer (such as the Model 403A available from Aurora Scientific Inc, Canada, or equivalent), as described in: Zhang, Z. et al. (1999) "Mechanical strength of single microcapsules determined by a novel micromanipulation technique." J. Microencapsulation, vol 16, no. 1, pages 117-124, and in: Sun, G. and Zhang, Z. (2001) "Mechanical Properties of Melamine-Formaldehyde microcapsules." J. Microencapsulation, vol 18, no. 5, pages 593-602, and as available at the University of Birmingham, Edgbaston, Birmingham, UK.
c.) A drop of the microcapsule suspension is placed onto a glass microscope slide, and dried under ambient conditions for several minutes to remove the water and achieve a sparse, single layer of solitary particles on the dry slide. Adjust the concentration of microcapsules in the suspension as needed to achieve a suitable particle density on the slide. More than one slide preparation may be needed.
d.) The slide is then placed on a sample-holding stage of the micromanipulation instrument. Thirty or more microcapsules on the slide(s) are selected for measurement, such that there are at least ten microcapsules selected within each of three pre-determined size bands. Each size band refers to the diameter of the microcapsules as derived from the Accusizer-generated volume-weighted PSD. The three size bands of particles are: the Mean Diameter +/- 2 µm; the 5^{th} Percentile Diameter +/- 2 µm; and the 90^{th} Percentile Diameter +/- 2 µm. Microcapsules which appear deflated, leaking or damaged are excluded from the selection process and are not measured.
e.) For each of the 30 or more selected microcapsules, the diameter of the microcapsule is measured from the image on the micromanipulator and recorded. That same microcapsule is then compressed between two flat surfaces, namely the flat-ended force probe and the glass microscope slide, at a speed of 2 µm per second, until the microcapsule is ruptured. During the compression step, the probe force is continuously measured and recorded by the data acquisition system of the micromanipulation instrument.
f.) The cross-sectional area is calculated for each of the microcapsules, using the diameter measured and assuming a spherical particle (πr², where r is the radius of the particle before compression). The rupture force is determined for each sample by reviewing the recorded force probe measurements. The measurement probe measures the force as a function of distance compressed. At one compression, the microcapsule ruptures and the measured force will abruptly stop. This maxima in the measured force is the rupture force.
g.) The Fracture Strength of each of the 30 or more microcapsules is calculated by dividing the rupture force (in Newtons) by the calculated cross-sectional area of the respective microcapsule.
h.) On a plot of microcapsule diameter versus fracture-strength, a Power Regression trendline is fit against all 30 or more raw data points, to create a modeled distribution of the relationship between microcapsule diameter and fracture-strength.
i.) The percentage of microcapsules which have a fracture strength value within a specific strength range is determined by viewing the modeled relationship plot to locate where the curve intersects the relevant fracture-strength limits, then reading off the microcapsule size limits corresponding with those strength limits. These microcapsule size limits are then located on the volume-weighted PSD plot and thus identify an area under the PSD curve which corresponds to the portion of microcapsules falling within the specified strength range.

The identified area under the PSD curve is then calculated as a percentage of the total area under the PSD curve. This percentage indicates the percentage of microcapsules falling with the specified range of fracture strengths.

## Claims

1. An anhydrous antiperspirant composition comprising:
a parent fragrance that is dispersed throughout the composition; and
a friction-triggered fragrance delivery technology comprising a plurality of microcapsules; wherein the microcapsules comprise a core material and a shell encapsulating the core material; wherein the core material comprises a first non-parent fragrance and the shell comprises a synthetic polymeric material; wherein the synthetic polymeric material comprises a polyacrylate material; wherein the microcapsules are spray-dried to form spray-dried microcapsules;
wherein the composition further comprises
a moisture-triggered fragrance delivery technology comprising a second non-parent fragrance; and
wherein the second non-parent fragrance is encapsulated in cyclodextrin particles.

2. The anhydrous antiperspirant composition or method according to claim 1, wherein the composition further comprises:
from 0.1% to 30% by weight of the composition, of one or more antiperspirant actives;
from 0.1% to 35% by weight of the composition, of one or more structurants; and
from 10% to 99% by weight of the composition, of one or more anhydrous liquid carriers; wherein the composition is an anhydrous composition.

3. The anhydrous antiperspirant composition according to any preceding claim, wherein the microcapsules have a fracture strength of from 0.2 mega Pascals to 10.0 mega Pascals, preferably from 0.2 mega Pascals to 2.0 mega Pascals, according to the Fracture Strength Test Method as disclosed herein.

4. The anhydrous antiperspirant composition according to any of the preceding claims, wherein the polyacrylate material can form at least 5% of the overall mass of the shell.

5. The anhydrous antiperspirant composition of claim 1, wherein the anhydrous antiperspirant composition is a solid anhydrous antiperspirant composition.

6. The anhydrous antiperspirant composition of claim 5, wherein the solid anhydrous antiperspirant composition further comprises a non-volatile organic fluid; wherein the concentration of the non-volatile organic fluid ranges from 1 % to 20%, by weight of the composition.

7. The anhydrous antiperspirant composition of claim 6, wherein the nonvolatile organic fluids are selected from the group consisting of mineral oil, PPG-14 butyl ether, isopropyl myristate, petrolatum, butyl stearate, cetyl octanoate, butyl myristate, myristyl myristate, e12-15 alkylbenzoate, dipropylene glycol dibenzoate, PPG-15 stearyl ether benzoate and blends thereof, neopentyl glycol diheptano ate , octyldodecanol, isostearyl isostearate, octododecyl benzoate, isostearyl lactate, isostearyl palmitate, isononyl/ isononoate, isoeicosane, octyldodecyl neopentanate, hydrogenated polyisobutane, and isobutyl stearate.

8. The anhydrous antiperspirant composition of claim 5, wherein the solid anhydrous antiperspirant composition further comprises a non-volatile silicone fluid; wherein the concentration of the non-volatile silicone is from 1 %, to 15%, by weight of the composition.

9. The anhydrous antiperspirant composition of any of the claims 5-8, wherein the antiperspirant active is selected from the group consisting of zirconium-containing salts or materials, such as zirconyl oxyhalides, zirconyl hydroxyhalides, and mixtures thereof; and/or aluminum-containing salts such as, for example, aluminum halides, aluminum chlorohydrate, aluminum hydroxyhalides, and mixtures thereof

10. The anhydrous antiperspirant composition of any of the claims 5-9, wherein the structurant is selected from waxes or wax-like materials having a melt point of above 65°C selected from beeswax, carnauba, bayberry, candelilla, montan, ozokerite, ceresin, hydrogenated castor oil (castor wax), synthetic waxes and microcrystalline waxes.

11. The anhydrous antiperspirant composition according to any of the claims 5-10, wherein the antiperspirant composition comprises a volatile silicone being a cyclic silicone.

12. The anhydrous antiperspirant composition of claim 11, wherein the volatile silicone conforms to the formula: 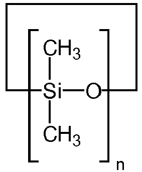 wherein n is from 3, or from 5; to 7, or 6.

## Patentansprüche

1. Wasserfreie, schweißhemmende Zusammensetzung, umfassend:
einen Stammduftstoff, der durch die Zusammensetzung dispergiert ist; und
eine durch Reibung auslösende Duftstoffabgabetechnologie, umfassend eine Vielzahl von Mikrokapseln; wobei die Mikrokapseln ein Kernmaterial und eine Schale, die das Kernmaterial einkapselt, umfassen; wobei das Kernmaterial einen ersten nicht-Stammduftstoff umfasst und die Schale ein synthetisches Polymermaterial umfasst; wobei das synthetische Polymermaterial ein Polyacrylatmaterial umfasst; wobei die Mikrokapseln sprühgetrocknet werden, um sprühgetrocknete Mikrokapseln zu bilden;
wobei die Zusammensetzung ferner eine durch Feuchtigkeit auslösende Duftstoffabgabetechnologie umfasst, die einen zweiten nicht-Stammduftstoff umfasst; und
wobei der zweite nicht-Stammduftstoff in Cyclodextrinteilchen eingekapselt ist.

2. Wasserfreie, schweißhemmende Zusammensetzung oder Verfahren nach Anspruch 1, wobei die Zusammensetzung ferner Folgendes umfasst:
zu 0,1 Gew.-% bis 30 Gew.-% der Zusammensetzung einen oder mehrere schweißhemmende Wirkstoffe;
zu 0,1 Gew.-% bis 35 Gew.-% der Zusammensetzung ein oder mehrere Strukturmittel; und
zu 10 Gew.-% bis 99 Gew.-% der Zusammensetzung einen oder mehrere wasserfreie, flüssige Träger; wobei die Zusammensetzung eine wasserfreie Zusammensetzung ist.

3. Wasserfreie, schweißhemmende Zusammensetzung nach einem der vorstehenden Ansprüche, wobei die Mikrokapseln gemäß dem hierin offenbarten Bruchfestigkeitsprüfverfahren eine Bruchfestigkeit von 0,2 Megapascal bis 10,0 Megapascal, vorzugsweise von 0,2 Megapascal bis 2,0 Megapascal, aufweisen.

4. Wasserfreie, schweißhemmende Zusammensetzung nach einem der vorstehenden Ansprüche, wobei das Polyacrylatmaterial wenigstens 5 % der Gesamtmasse der Schale bilden kann.

5. Wasserfreie, schweißhemmende Zusammensetzung nach Anspruch 1, wobei die wasserfreie, schweißhemmende Zusammensetzung eine feste, wasserfreie, schweißhemmende Zusammensetzung ist.

6. Wasserfreie, schweißhemmende Zusammensetzung nach Anspruch 5, wobei die feste, wasserfreie, schweißhemmende Zusammensetzung ferner ein nichtflüchtiges organisches Fluid umfasst; wobei die Konzentration des nichtflüchtigen organischen Fluids im Bereich von 1 Gew.-% bis 20 Gew.-% der Zusammensetzung liegt.

7. Wasserfreie, schweißhemmende Zusammensetzung nach Anspruch 6, wobei die nichtflüchtigen organischen Fluide ausgewählt sind aus der Gruppe bestehend aus Mineralöl, PPG-14-butylether, Isopropylmyristat, Petrolatum, Butylstearat, Cetyloctanoat, Butylmyristat, Myristylmyristat, e12-15-Alkylbenzoat, Dipropylenglycoldibenzoat, PPG-15-stearyletherbenzoat und Gemischen davon, Neopentylglycoldiheptanoat, Octyldodecanol, Isostearylisostearat, Octododecylbenzoat, Isostearyllactat, Isostearylpalmitat, Isononylisononoat, Isoeicosan, Octyldodecylneopentanat, hydriertem Polyisobutan und Isobutylstearat.

8. Wasserfreie, schweißhemmende Zusammensetzung nach Anspruch 5, wobei die feste, wasserfreie, schweißhemmende Zusammensetzung ferner ein nichtflüchtiges Silikonfluid umfasst; wobei die Konzentration des nichtflüchtigen Silikons von 1 Gew.-% bis 15 Gew.-% der Zusammensetzung beträgt.

9. Wasserfreie, schweißhemmende Zusammensetzung nach einem der Ansprüche 5 bis 8, wobei der schweißhemmende Wirkstoff ausgewählt ist aus der Gruppe, bestehend aus zirkoniumhaltigen Salzen, wie Zirkonyloxyhalogeniden, Zirkonylhydroxyhalogeniden und Mischungen davon; und/oder aluminiumhaltigen Salzen wie zum Beispiel Aluminiumhalogeniden, Aluminiumchlorhydrat, Aluminiumhydroxyhalogeniden und Mischungen davon.

10. Wasserfreie, schweißhemmende Zusammensetzung nach einem der Ansprüche 5 bis 9, wobei das Strukturmittel ausgewählt ist aus Wachsen oder wachsähnlichen Materialien mit einem Schmelzpunkt von über 65 °C, ausgewählt aus Bienenwachs, Carnauba, Gagelstrauch, Kandelilla, Montan, Ozokerit, Ceresin, hydriertem Rizinusöl (Rizinuswachs), synthetischen Wachsen und mikrokristallinen Wachsen.

11. Wasserfreie, schweißhemmende Zusammensetzung nach einem der Ansprüche 5 bis 10, wobei die schweißhemmende Zusammensetzung ein flüchtiges Silikon umfasst, das ein cyclisches Silikon ist.

12. Wasserfreie, schweißhemmende Zusammensetzung nach Anspruch 11, wobei das flüchtige Silikon der folgenden Formel entspricht: 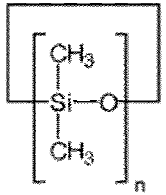 worin n von 3 oder von 5; bis 7 oder 6 ist.

## Revendications

1. Composition anti-transpiration anhydre comprenant :
un parfum parent qui est dispersé sur l'ensemble de la composition ; et
une technologie de libération de parfum déclenchée par frottement comprenant une pluralité de microgélules ; dans laquelle les microgélules comprennent un matériau de noyau et une coque encapsulant le matériau de noyau ; dans laquelle le matériau de noyau comprend un premier parfum non parent et la coque comprend un matériau polymère synthétique ; dans laquelle le matériau polymère synthétique comprend un matériau de polyacrylate ; dans laquelle les microgélules sont séchées par atomisation pour former des microgélules séchées par atomisation ;
où la composition comprend en outre une technologie de libération de parfum déclenchée par humidité comprenant un deuxième parfum non parent ; et
dans laquelle le deuxième parfum non parent est encapsulé dans des particules de cyclodextrine.

2. Composition anti-transpiration anhydre ou procédé selon la revendication 1, où la composition comprend en outre :
de 0,1 % à 30 % en poids de la composition, d'un ou plusieurs agents actifs anti-transpiration ;
de 0,1 % à 35 % en poids de la composition, d'un ou plusieurs structurants ; et
de 10 % à 99 % en poids de la composition, d'un ou plusieurs véhicules liquides anhydres ; où la composition est une composition anhydre.

3. Composition anti-transpiration anhydre selon l'une quelconque revendication précédente, dans laquelle les microgélules ont une résistance à la rupture allant de 0,2 mégapascal à 10,0 mégapascals, de préférence de 0,2 mégapascal à 2,0 mégapascals, selon le procédé de test de résistance à la rupture tel que décrit ici.

4. Composition anti-transpiration anhydre selon l'une quelconque des revendications précédentes, dans laquelle le matériau de polyacrylate peut former au moins 5 % de la masse globale de la coque.

5. Composition anti-transpiration anhydre selon la revendication 1, où la composition anti-transpiration anhydre est une composition anti-transpiration anhydre solide.

6. Composition anti-transpiration anhydre selon la revendication 5, où la composition anti-transpiration anhydre solide comprend en outre un fluide organique non volatil ; dans laquelle la concentration du fluide organique non volatil va de 1 % à 20 % en poids de la composition.

7. Composition anti-transpiration anhydre selon la revendication 6, dans laquelle les fluides organiques non volatils sont choisis dans le groupe constitué d'huile minérale, éther butylique de PPG-14, myristate d'isopropyle, vaseline, stéarate de butyle, octanoate de cétyle, myristate de butyle, myristate de myristyle, e12-15 alkylbenzoate, dibenzoate de dipropylène glycol, benzoate d'éther stéarylique de PPG-15 et des mélanges de celui-ci, diheptanoate de néopentyl-glycol, octyldodécanol, isostéarate d'isostéaryle, benzoate d'octododécyle, lactate d'isostéaryle, palmitate d'isostéaryle, isononoate d'isononyle/, isoeicosane, néopentanoate d'octyldodécyle, polyisobutane hydrogéné, et stéarate d'isobutyle.

8. Composition anti-transpiration anhydre selon la revendication 5, où la composition anti-transpiration anhydre solide comprend en outre un fluide siliconé non volatil ; dans laquelle la concentration de la silicone non volatile va de 1 % à 15 % en poids de la composition.

9. Composition anti-transpiration anhydre selon l'une quelconque des revendications 5 à 8, dans laquelle l'agent actif anti-transpiration est choisi dans le groupe constitué de sels ou matériaux contenant du zirconium, tels que des oxyhalogénures de zirconyle, des hydroxyhalogénures de zirconyle, et leurs mélanges ; et/ou des sels contenant de l'aluminium tels que, par exemple, des halogénures d'aluminium, du chlorhydrate d'aluminium, des hydroxyhalogénures d'aluminium, et leurs mélanges.

10. Composition anti-transpiration anhydre selon l'une quelconque des revendications 5 à 9, dans laquelle le structurant est choisi parmi des cires ou des matériaux de type cire possédant un point de fusion supérieur à 65 °C choisis parmi la cire d'abeille, le carnauba, la baie de laurier, la cire de candililla, la cire de lignite, l'ozocérite, la cérésine, l'huile de ricin hydrogénée (cire de ricin), des cires synthétiques et des cires microcristallines.

11. Composition anti-transpiration anhydre selon l'une quelconque des revendications 5 à 10, où la composition anti-transpiration comprend une silicone volatile qui est une silicone cyclique.

12. Composition anti-transpiration anhydre selon la revendication 11, dans laquelle la silicone volatile répond à la formule : 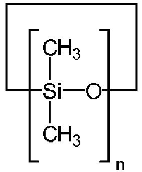 dans laquelle n va de 3, ou de 5 ; à 7, ou 6.
